# EUROPEAN PATENT SPECIFICATION

(11) **EP 4 275 744 B1**
(45) Date of publication and mention of the grant of the patent: **01.10.2025**
(21) Application number: 22904569.5
(22) Date of filing: 02.12.2022
(51) Int. Cl.: A61N 7/02, A61B 8/08, A61B 8/00, A61N 7/00

(54) **ULTRASOUND IMAGING APPARATUS FOR BRAIN DISEASE TREATMENT AND ULTRASOUND IMAGING AND TREATMENT METHOD USING SAME**
ULTRASCHALLBILDGEBUNGSVORRICHTUNG ZUR BEHANDLUNG VON HIRNERKRANKUNGEN UND ULTRASCHALLBILDGEBUNGS- UND -BEHANDLUNGSVERFAHREN DAMIT
APPAREIL D'IMAGERIE ULTRASONORE POUR LE TRAITEMENT D'UNE MALADIE CÉRÉBRALE ET PROCÉDÉ D'IMAGERIE ULTRASONORE ET DE TRAITEMENT L'UTILISANT

(30) Priority: 08.12.2021 KR 20210175190
(43) Date of publication of application: 15.11.2023
(73) Proprietor: IMGT Co, Ltd., Seongnam-si, Gyeonggi-do 13605 (KR)
(72) Inventor: SON, Keon Ho, Seongnam-si Gyeonggi-do 13462 (KR); KIM, Dae Seung, Seoul 07524 (KR)
(74) Representative: Sander, Rolf
(86) International application number: PCT/KR2022/019444
(87) International publication number: WO 2023/106739

(56) References cited:
- KR-A- 20070 105 607
- KR-A- 20170 084 945
- KR-A- 20190 097 165
- KR-A- 20200 059 096
- KR-A- 20200 108 130
- US-A1- 2016 317 129
- US-A1- 2019 184 204

## Description

### [Technical Field]

The present invention relates to diagnosis and treatment technology using ultrasound, and more particularly, to imaging and treatment technology using focused ultrasound (FUS) for image guided therapy.

The present invention is derived from research carried out as part of a study conducted as part of Collaborative Life-Cycle Medical Device R&D Project of the Ministry of Science and Information & Communication Technology, the Ministry of Trade, Industry, and Energy, the Ministry of Health and Welfare, and the Ministry of Food and Drug Safety [Project number: 9991006682, KMDF_PR_20200901_0009, research project name: Development of Commercialization of Market-Leading Ultrasound Image-Guide, High-Intensity Focused Ultrasound Treatment Device for Integrated Therapy for Pancreatic Cancer, research management institution: Korea Medical Device Development Fund, contribution rate: 100%, research supervising institute: IMGT. Co., Ltd., research period: March 1, 2022-December 31, 2022].

### [Background Art]

Ultrasound signals may be used in the treatment of biological tissues, such as cancer, tumors, lesions, and the like. Treatment with ultrasound is a method of treating a lesion by emitting ultrasound signals to the lesion of the human body. Ultrasound treatment may cause less trauma of a patient, compared to general surgical treatment or chemotherapy, and realize non-invasive treatment. Examples of the application of ultrasound treatment include liver cancer, bone sarcoma, breast cancer, pancreatic cancer, kidney cancer, soft tissue tumors, pelvic tumors, and the like.

The patent publication US 2016/317129 A1 relates to an integrated headset for ultrasound monitoring and therapy.

### [Disclosure]

### [Technical problem]

A patient-friendly, miniaturized, and low-cost ultrasound imaging apparatus for non-invasive brain disease treatment and an ultrasound imaging and treatment method using the same are proposed.

### [Technical Solution]

An ultrasound imaging apparatus for brain disease treatment according to the invention includes the features of claim 1.

The medical image may be an image of the brain that is any one of a magnetic resonance (MR) image, a computed tomography (CT) image, a positron emission tomography (PET) image, and a PET-CT image captured respectively through a magnetic resonance imaging (MRI) device, a CT device, a PET device, and a PET-CT device.

The transducer unit may include an ultrasound array transducer configured to perform both imaging of the image of the brain of the patient and ultrasound focusing of a brain lesion.

The image generating unit may generate a diagnostic ultrasound focused image on the basis of a diagnostic ultrasound signal received from the brain, and the position alignment unit may align a focal position of the generated diagnostic ultrasound focused image with a position of a lesion in the obtained medical image.

The transducer unit includes an imaging transducer configured to transmit and receive a diagnostic ultrasound signal for imaging an image of the brain of the patient, and a treatment transducer configured to transmit a focused ultrasound signal to a brain lesion.

The imaging transducer may be a phased array imaging transcranial transducer.

The image generation unit may generate a transcranial ultrasound image on the basis of a diagnostic ultrasound signal received from the brain, and the position alignment unit may align a position of the generated transcranial ultrasound image with a position of a lesion in the obtained medical image.

### [Advantageous Effects]

According to an ultrasound imaging apparatus for brain disease treatment and an ultrasound imaging and treatment method using the same, it is possible to non-invasively treat brain disease using ultrasound image-guided focused ultrasound.

The ultrasound imaging apparatus is patient-friendly, miniaturized, cost-effective, and thus applicable to general treatment. For example, it may be usefully used even in small- and medium-sized hospitals. In particular, since the ultrasound imaging apparatus uses pre-captured medical images such as magnetic resonance (MR) images, it is not necessary to obtain medical images through MR image capture in real-time, and thus miniaturization and cost reduction may be achieved.

The ultrasound imaging apparatus matches a pre-captured medical image with a diagnostic ultrasound focus image, or a transcranial ultrasound image obtained using a transcranial transducer enabling non-invasive image diagnosis, and performs diagnosis and treatment using a matched image, thereby allowing for accurate treatment for brain disease.

By using a medical headset, the medical image may be mechanically matched with the diagnostic ultrasound focused image or the transcranial ultrasound image and the focus depth for focusing ultrasound to a treatment site may be adjusted after the matching.

### [Description of Drawings]

FIG. 1 is a block diagram illustrating the configuration of an ultrasound imaging apparatus for brain disease treatment (hereinafter simply referred to as an "ultrasound imaging apparatus) according to the present invention;
FIG. 2 is a diagram illustrating the structure of an ultrasound transducer unit according to the present invention;
FIG. 3 is a diagram illustrating the structure of a position alignment unit according to the present invention;
FIG. 4 is a diagram illustrating an example of matching a medical image and an ultrasound image according to the present invention;
FIG. 5 is a diagram illustrating an appearance of an ultrasound imaging apparatus according to the present invention;
FIG. 6 is a diagram illustrating the structure of an ultrasound transducer unit and a position alignment unit according to the present invention; and
FIG. 7 is a diagram illustrating an example of matching a medical image and an ultrasound image according to the present invention.

### [Modes of the Invention]

The advantages and features of the present invention and the manner of achieving the advantages and features will become apparent with reference to the details below together with the accompanying drawings. The same reference numerals refer to the same components throughout this disclosure.

Combinations of each block of the accompanying block diagram and each step of the flowchart may be executed by computer program instructions (execution engine), and these computer program instructions are executed by a processor of a general-purpose computer, special-purpose computer, or other programmable data processing device. Since it can be mounted, the instructions executed through the processor of a computer or other programmable data processing device create means for performing the functions described in each block of the block diagram or each step of the flowchart.

These computer program instructions may also be stored in a computer usable or computer readable memory that can be directed to a computer or other programmable data processing device to implement functionality in a particular way, such that the computer usable or computer readable memory the instructions stored in are also capable of producing an article of manufacture containing instruction means for performing the functions described in each block of the block diagram or each step of the flow chart.

And since the computer program instructions can also be loaded on a computer or other programmable data processing device, a series of operational steps are performed on the computer or other programmable data processing device to create a computer-executed process to create a computer or other programmable data processing device. It is also possible that the instructions for performing the data processing apparatus provide steps for executing the functions described in each block of the block diagram and each step of the flowchart.

Further, each block or each step may represent a module, a segment, or a part of a code, which includes one or more executable instructions for performing specified logical functions, and it should be noted that, in some alternative embodiments, the functions described in the blocks or steps may occur out of sequence. For example, two blocks or steps shown in succession may in fact be substantially executed at the same time, and the two blocks or steps may also be executed in the reverse order of the corresponding function as necessary.

Hereinafter, embodiments of the present invention will be described in detail with reference to the accompanying drawings.

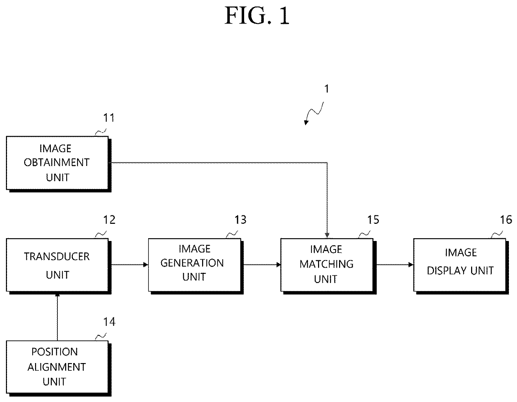

FIG. 1 is a block diagram illustrating the configuration of an ultrasound imaging apparatus for brain disease treatment (hereinafter simply referred to as an "ultrasound imaging apparatus") according to the present invention.

An ultrasound imaging apparatus 1 is an apparatus for non-invasive treatment of brain diseases using ultrasound image-guided focused ultrasound (FUS).

Referring to FIG. 1, the ultrasound imaging apparatus 1 includes an image obtainment unit 11, a transducer unit 12, an image generation unit 13, a position alignment unit 14, an image matching unit 15, and an image display unit 16.

The image obtainment unit 11 obtains a medical image of the brain of a patient captured before a procedure. In this case, the medical image may be an image of the brain that is any one of a magnetic resonance (MR) image, a computed tomography (CT) image, a positron emission tomography (PET) image, and a PET-CT image captured respectively through a magnetic resonance imaging (MRI) device, a CT device, a PET device, and a PET-CT device. The above-described medical image may be an image of the patient captured within a preset period, and may be used as valid data because there is no significant change in data within the preset period. In addition, since there is no need to capture a medical image in real-time, cost reduction and the miniaturization of the apparatus 1 may be achieved.

The transducer unit 12 transmits an ultrasound signal for imaging and ultrasound focusing of the brain of the patient.

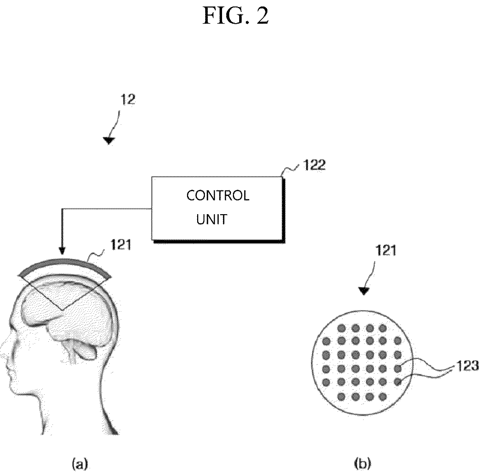

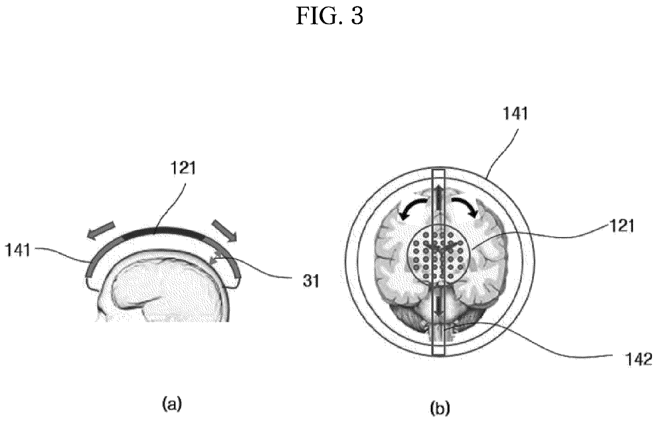

The transducer unit 12 may be an ultrasound array transducer configured to perform both imaging of the image of the patient's brain and ultrasound focusing of a brain lesion. For example, the transducer unit 12 may both receive a diagnostic ultrasound signal and transmit a focused ultrasound signal. An embodiment of this will be described below with reference to FIGS. 2 and 3.

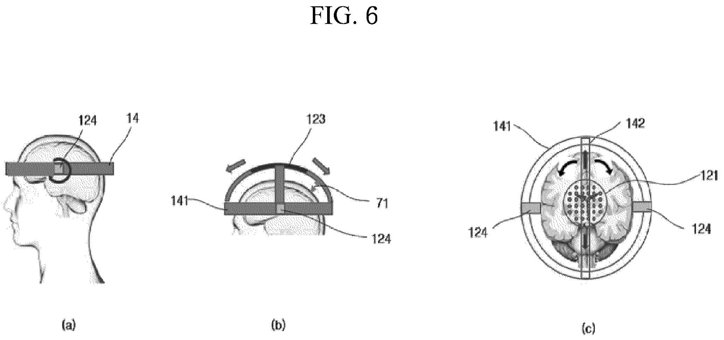

The transducer unit 12 may include an imaging transducer and a treatment transducer completely separated from the imaging transducer. The imaging transducer transmits and receives a diagnostic ultrasound signal for imaging the image of the brain of the patient. The treatment transducer transmits a focused ultrasound signal to a brain lesion for the purpose of treatment of brain disease. In this case, the imaging transducer may be a phased array imaging transcranial transducer. An embodiment of this will be described below with reference to FIG. 6.

The image generation unit 13 generates an ultrasound image on the basis of an ultrasound signal received from the brain. The position alignment unit 14 aligns the position of the ultrasound image with the position of the medical image obtained through the image obtainment unit 11 by controlling the position of the transducer unit 12

The image generation unit 13 may generate a diagnostic ultrasound focused image on the basis of a diagnostic ultrasound signal received from the brain, and the position alignment unit 14 may align a focal position of the generated diagnostic ultrasound focused image with the position of the obtained medical image.

The image generation unit 13 may generate a transcranial ultrasound image on the basis of the diagnostic ultrasound signal received from the brain, and the position alignment unit 14 may align the position of the generated transcranial ultrasound image with the position of the obtained medical image.

The position alignment unit 14 may align the positions by a stereotaxic method using a medical headset. In this case, the medical headset may move the ultrasound transducer unit 12 to a predetermined position such that a position of an ultrasound image obtained through the ultrasound transducer unit 12 coincides with the position of the medical image. To this end, the medical headset may include a tiltable and rotatable device.

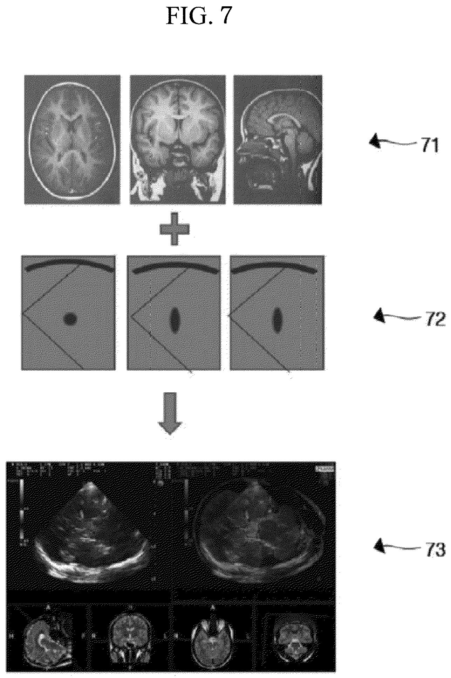

The position alignment unit 14 may include a driving unit configured to change an ultrasound focusing area of the ultrasound transducer unit 12. In this case, the driving unit may change the ultrasound focusing area of the ultrasound transducer unit 12 by driving the tiltable and rotatable device. For example, the position alignment unit 14 may adjust a focus depth. An example of changing the ultrasound focusing area will be described below with reference to FIGS. 3 and 7.

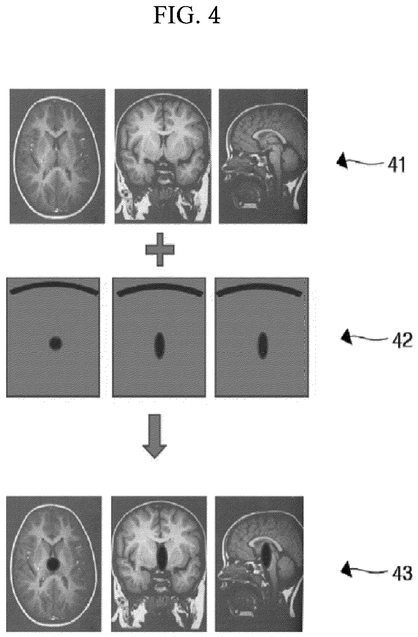

The image matching unit 15 matches two images of which positions are matched through the position alignment unit 14. An embodiment of image matching will be described with reference to FIGS. 4 and 7.

The image display unit 16 detects and displays a brain lesion using a matched image.

FIG. 2 is a diagram illustrating the structure of an ultrasound transducer unit according to the present invention.

Referring to FIGS. 1 and 2, the ultrasound transducer unit 12 includes an ultrasound array transducer 121 and a control unit 122.

In detail, (a) of FIG. 2 is a side view of the ultrasound array transducer 121, and (b) is a plan view of the ultrasound array transducer 121.

The ultrasound array transducer 121 may use one transducer to perform both imaging of the image of the brain of the patient and ultrasound focusing of a brain lesion. In this case, the ultrasound array transducer 121 may both transmission Tx and reception Rx. For example, the ultrasound array transducer 121 may both receive a diagnostic ultrasound signal and transmit a focused ultrasound signal. For example, one ultrasound array transducer 121 may transmit a focused ultrasound signal of 250 kHz and a diagnostic ultrasound signal of 750 kHz, as well as receive a diagnostic ultrasound signal of 750 kHz.

The ultrasound array transducer 121 may include a plurality of sub-arrays 123, and each sub-array 123 includes an acoustic emission surface and a plurality of transducer elements. The control unit 122 may provide a driving signal to the plurality of transducer elements, and in response to the driving signal of the control unit 122, the plurality of transducer elements may emit acoustic energy from their respective acoustic emission surface areas to remove the patient's brain lesion.

FIG. 3 is a diagram illustrating the structure of a position alignment unit according to the present invention.

In more detail, (a) of FIG. 3 is a side view focusing on the position alignment unit 14, and (b) is a top plan view.

Referring to FIGS. 1 and 3, the position alignment unit 14 may be in the form of a medical headset 141 worn on the head of the patient. In this case, the position alignment unit 14 may use the medical headset 141 to move the ultrasound transducer unit 12 to a predetermined position such that the position of the ultrasound image obtained through the ultrasound transducer unit 12 coincides with the position of the medical image. To this end, the position alignment unit 14 may include a tiltable and rotatable device 142 and a driving unit (not shown) configured to drive the device 142, and the driving unit may move the position of the ultrasound transducer unit 12 by driving the device 142. The driving unit operates under the control of the control unit 122.

The position alignment unit 14 may change an ultrasound focusing area using the medical headset 141. For example, the position alignment unit 14 may adjust a focus depth 31 by position adjustment of the ultrasound transducer unit 12 through the driving unit, thereby changing the ultrasound focusing area.

FIG. 4 is a diagram illustrating an example of matching a medical image and an ultrasound image according to an embodiment of the present invention.

Referring to FIGS. 1 and 4, the image matching unit 15 generates a matched image 43 by matching a medical image 42 and an ultrasound focused image 42 whose positions are aligned through the position alignment unit 14. The ultrasound focused image 42 may be an image generated using an ultrasound signal obtained using the ultrasound array transducer 121 of FIG. 2.

Although FIG. 4 shows an MR image as an example of the medical image 41, the medical image 41 may be a CT image, a PET image, or a PET-CT image.

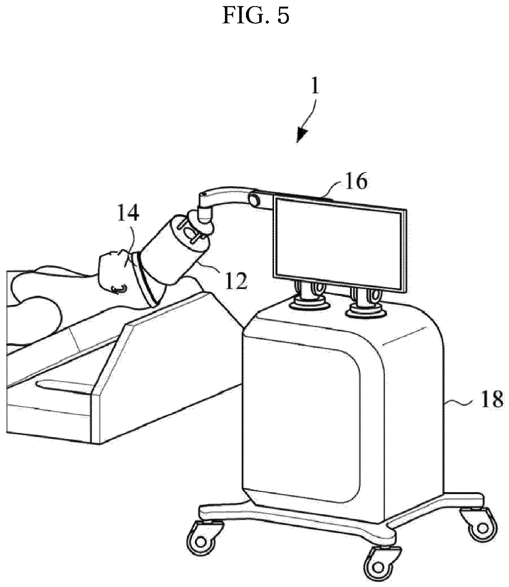

FIG. 5 is a diagram illustrating an appearance of an ultrasound imaging apparatus according to the present invention.

Referring to FIGS. 1 and 5, the ultrasound imaging apparatus 1 is provided with an ultrasound transducer unit 12 and an image display unit 16 in a main body 18. The position alignment unit 14 may be mounted on the head area of the patient in the form of a medical headset. The ultrasound transducer unit 12 may be in the form of an ultrasound therapeutic head as shown in FIG. 5.

FIG. 6 is a diagram illustrating the structure of an ultrasound transducer unit and a position alignment unit according to the present invention.

In more detail, (a) is a side view focusing on the imaging transducer in the ultrasound transducer unit, (b) is a side view focusing on the position alignment unit, and (c) is a top plan view focusing on the position alignment unit.

Referring to FIGS. 1 and 6, the ultrasound transducer unit 12 has a treatment transducer 123 and an imaging transducer 124 completely separated from the treatment transducer 123.

The treatment transducer 123 is configured to emit focused ultrasound for treating a patient. The treatment transducer 123 may have a focused ultrasound emission surface. The treatment transducer 123 generates a focused ultrasound signal and focuses the focused ultrasound signal to a treatment site in the brain. The treatment transducer 123 may have an array structure composed of a plurality of treatment transducers and the plurality of treatment transducers constituting the array may be arranged in a random form.

The imaging transducer 124 is provided to obtain a medical image of the brain. An operator may perform ultrasound treatment using the treatment transducer 123 while checking the medical image obtained by the imaging transducer 124. The imaging transducer 124 may be configured to transmit an ultrasound signal to the patient's brain and receive an ultrasound signal reflected from the brain. For example, the imaging transducer 124 may be configured to have a piezoelectric element in a cylindrical-shaped casing.

The imaging transducer 12 may be a phased array imaging transcranial transducer as shown in FIG. 6. The transcranial transducer allows the ultrasound transducer, which generates ultrasound, to be in close contact with and supported by the skull of a human body so as to transmit ultrasound to transcranial. The skull of the human body may be formed in various sizes and shapes according to race, age, and sex, and an affected part, that is, a position of the brain, to which ultrasound needs to be transmitted, may also vary. Therefore, the position alignment unit 14 may adjust the position of the imaging transducer 124 so that the imaging transducer 124 can be constantly in close contact with and supported by the skull according to the shape and size of the skull and the position of the brain to which ultrasound needs to be transmitted.

The position alignment unit 14 may be in the form of a medical headset 141 worn on the head of the patient. In this case, the position alignment unit 14 may use the medical headset 141 to move the imaging transducer 124 to a predetermined position such that the position of the ultrasound image obtained through the imaging transducer 124 coincides with the position of the medical image. To this end, the position alignment unit 14 may include a tiltable and rotatable device 142 and a driving unit (not shown) configured to drive the device 142, and the driving unit may move the position of the imaging transducer 124 by driving the device 142.

The position alignment unit 14 may change an ultrasound focusing area using the medical headset 141. For example, the position alignment unit 14 may adjust a focus depth 71 by position adjustment of the treatment transducer 123 through the driving unit, thereby changing the ultrasound focusing area.

FIG. 7 is a diagram illustrating an example of matching a medical image and an ultrasound image according to the present invention.

Referring to FIGS. 1, 6, and 7, the imaging transducer 124 may be a transcranial transducer, and the image generation unit 13 generates a transcranial ultrasound image on the basis of a diagnostic ultrasound signal received from the patient's brain. The position alignment unit 14 may align the position of the generated transcranial ultrasound image with the position of the obtained medical image.

The position alignment unit 14 may use the medical headset 141 of FIG. 6 to move the imaging transducer 124 to a predetermined position such that the position of the ultrasound image obtained through the imaging transducer 124 coincides with the position of the medical image.

The image matching unit 15 generates a matched image 73 by matching a medical image 71 and a transcranial ultrasound image 72 whose positions are aligned through the position alignment unit 14. Although FIG. 7 shows an MR image as an example of the medical image 71, the medical image 71 may be a CT image, a PET image, or a PET-CT image.

After the matching, the position aligning unit 14 may change the ultrasound focusing area using the medical headset 141.

## Claims

1. An ultrasound imaging apparatus (1) for brain disease treatment, comprising:
an image obtainment unit (11) which obtains a medical image of the brain of a patient captured before a procedure;
a transducer unit (12) which transmits an ultrasound signal for imaging and ultrasound focusing of the brain of the patient;
an image generation unit (13) which generates an ultrasound image on the basis of the ultrasound signal received from the brain;
a position alignment unit (14) which mechanically controls a position of the transducer unit to align a position of the generated ultrasound image with a position of the obtained medical image;
an image matching unit (15) configured to generate a matched image by matching the obtained medical image and the generated ultrasound image whose positions are aligned through the position alignment unit; and
an image display unit (16) which detects and displays a brain lesion by using the matched image, wherein the position alignment unit (14) comprises a medical headset (141) worn on the head of the patient and **characterized in that**:
the medical headset (141) is configured to move the ultrasound transducer unit (12) to a predetermined position such that a position of the ultrasound image obtained through the ultrasound transducer unit (12) coincides with a position of a lesion in the medical image,
wherein the medical headset (141) comprises a tiltable and rotatable device (142) and a driving unit configured to drive the device (142), and
wherein the position alignment unit (14) is configured to change an ultrasound focusing area by adjusting a focus depth (31) through position adjustment of the ultrasound transducer unit (12).

2. The ultrasound imaging apparatus of claim 1, wherein the medical image is an image of the brain that is any one of a magnetic resonance (MR) image, a computed tomography (CT) image, a positron emission tomography (PET) image, and a PET-CT image captured respectively through a magnetic resonance imaging (MRI) device, a CT device, a PET device, and a PET-CT device.

3. The ultrasound imaging apparatus (1) of claim 1, wherein the transducer unit (12) comprises an ultrasound array transducer (121) configured to perform both imaging of the image of the brain of the patient and ultrasound focusing of a brain lesion.

4. The ultrasound imaging apparatus (1) of claim 3, wherein
the image generating unit (13) is configured to generate a diagnostic ultrasound focused image on the basis of a diagnostic ultrasound signal received from the brain and
the position alignment unit (14) is configured to align a focal position of the generated diagnostic ultrasound focused image with a position of a lesion in the obtained medical image.

5. The ultrasound imaging apparatus (1) of claim 1, wherein the transducer unit (12) comprises an imaging transducer (124) configured to transmit and receive a diagnostic ultrasound signal for imaging an image of the brain of the patient and a treatment transducer (123) configured to transmit a focused ultrasound signal to a brain lesion.

6. The ultrasound imaging apparatus (1) of claim 5, wherein the imaging transducer is a phased array imaging transcranial transducer.

7. The ultrasound imaging apparatus (1) of claim 5, wherein the image generation unit (13) is configured to generate a transcranial ultrasound image on the basis of a diagnostic ultrasound signal received from the brain and the position alignment unit is configured to align a position of the generated transcranial ultrasound image with a position of a lesion in the obtained medical image.

## Patentansprüche

1. Ultraschallbildgebungsvorrichtung (1) zur Behandlung von Hirnerkrankungen, umfassend:
eine Bilderhaltungseinheit (11), die ein medizinisches Bild des Hirns eines Patienten, das vor einem Vorgang erfasst wird, erhält;
eine Wandlereinheit (12), die ein Ultraschallsignal zur Bildgebung und Ultraschallfokussierung des Hirns des Patienten überträgt;
eine Bilderzeugungseinheit (13), die ein Ultraschallbild auf der Basis des Ultraschallsignals, das von dem Hirn empfangen wird, erzeugt;
eine Positionsausrichtungseinheit (14), die eine Position der Wandlereinheit mechanisch steuert, um eine Position des erzeugten Ultraschallbildes mit einer Position des erhaltenen medizinischen Bildes auszurichten;
eine Bildanpassungseinheit (15), die konfiguriert ist, um ein angepasstes Bild zu erzeugen, indem das erhaltene medizinische Bild und das erzeugte Ultraschallbild, deren Positionen durch die Positionsausrichteinheit ausgerichtet sind, angepasst werden; und
eine Bildanzeigeeinheit (16), die eine Hirnläsion durch Verwenden des angepassten Bildes detektiert und anzeigt,
wobei die Positionsausrichtungseinheit (14) ein medizinisches Headset (141) umfasst, das an dem Kopf des Patienten getragen wird, und
**dadurch gekennzeichnet, dass**:
das medizinische Headset (141) konfiguriert ist, um die Ultraschallwandlereinheit (12) zu einer vorbestimmten Position zu bewegen, sodass eine Position des Ultraschallbildes, das durch die Ultraschallwandlereinheit (12) erhalten wird, mit einer Position einer Läsion in dem medizinischen Bild zusammenfällt,
wobei das medizinische Headset (141) ein kippbares und drehbares Gerät (142) und eine Antriebseinheit, die konfiguriert ist, um das Gerät (142) anzutreiben, umfasst, und
wobei die Positionsausrichtungseinheit (14) konfiguriert ist, um einen Ultraschallfokussierungsbereich durch Einstellen einer Fokustiefe (31) durch Positionseinstellung der Ultraschallwandlereinheit (12) zu ändern.

2. Ultraschallbildgebungsvorrichtung nach Anspruch 1, wobei das medizinische Bild ein Bild des Hirns ist, das ein beliebiges von einem Magnetresonanz(MR-)Bild, einem Computertomographie(CT-)Bild, einem Positronenemissionstomographie(PET-)Bild und einem PET-CT-Bild ist, die jeweils durch ein Magnetresonanzbildgebungs(MRI-)Gerät, ein CT-Gerät, ein PET-Gerät und ein PET-CT-Gerät aufgenommen werden.

3. Ultraschallbildgebungsvorrichtung (1) nach Anspruch 1, wobei die Wandlereinheit (12) einen Ultraschallarray-Wandler (121) umfasst, der konfiguriert ist, um sowohl Bildgebung des Bildes des Hirns des Patienten als auch Ultraschallfokussierung einer Hirnläsion durchzuführen.

4. Ultraschallbildgebungsvorrichtung (1) nach Anspruch 3, wobei
die Bilderzeugungseinheit (13) konfiguriert ist, um ein diagnostisches fokussiertes Ultraschallbild auf der Basis eines diagnostischen Ultraschallsignals zu erzeugen, das von dem Hirn empfangen wird, und
die Positionsausrichtungseinheit (14) konfiguriert ist, um eine fokale Position des erzeugten diagnostischen fokussierten Ultraschallbildes mit einer Position einer Läsion in dem erhaltenen medizinischen Bild auszurichten.

5. Ultraschallbildgebungsvorrichtung (1) nach Anspruch 1, wobei die Wandlereinheit (12) einen Bildgebungswandler (124), der konfiguriert ist, um ein diagnostisches Ultraschallsignal zur Bildgebung eines Bildes des Hirns des Patienten zu übertragen und zu empfangen, und einen Behandlungswandler (123), der konfiguriert ist, um ein fokussiertes Ultraschallsignal zu einer Hirnläsion zu übertragen, umfasst.

6. Ultraschallbildgebungsvorrichtung (1) nach Anspruch 5, wobei der Bildgebungswandler ein transkranieller Phased-Array-Bildgebungswandler ist.

7. Ultraschallbildgebungsvorrichtung (1) nach Anspruch 5, wobei die Bilderzeugungseinheit (13) konfiguriert ist, um ein transkranielles Ultraschallbild auf der Basis eines diagnostischen Ultraschallsignals zu erzeugen, das von dem Hirn empfangen wird, und die Positionsausrichtungseinheit konfiguriert ist, um eine Position des erzeugten transkraniellen Ultraschallbildes mit einer Position einer Läsion in dem erhaltenen medizinischen Bild auszurichten.

## Revendications

1. Appareil d'imagerie ultrasonore (1) pour le traitement d'une maladie cérébrale, comprenant :
une unité d'obtention d'image (11) qui obtient une image médicale du cerveau d'un patient capturée avant une intervention ;
une unité de transducteurs (12) qui transmet un signal ultrasonore pour l'imagerie et la focalisation ultrasonore du cerveau du patient ;
une unité de génération d'image (13) qui génère une image ultrasonore sur la base du signal ultrasonore reçu du cerveau ;
une unité d'alignement de position (14) qui commande mécaniquement une position de l'unité de transducteurs pour aligner une position de l'image ultrasonore générée avec une position de l'image médicale obtenue ;
une unité d'appariement d'image (15) configurée pour générer une image appariée en appariant l'image médicale obtenue et l'image ultrasonore générée dont les positions sont alignées au moyen de l'unité d'alignement de position ;
une unité d'affichage d'image (16) qui détecte et affiche une lésion cérébrale à l'aide de l'image appariée,
ladite unité d'alignement de position (14) comprenant un casque médical (141) porté sur la tête du patient et **caractérisé en ce que** :
le casque médical (141) est configuré pour déplacer l'unité de transducteurs ultrasonores (12) vers une position prédéfinie de sorte qu'une position de l'image ultrasonore obtenue au moyen de l'unité de transducteurs ultrasonores (12) coïncide avec une position d'une lésion dans l'image médicale,
ledit casque médical (141) comprenant un dispositif inclinable et rotatif (142) et une unité d'entraînement configurée pour entraîner le dispositif (142), et
ladite unité d'alignement de position (14) étant configurée pour changer une zone de focalisation d'ultrasons en ajustant une profondeur de focalisation (31) par le biais d'un ajustement de position de l'unité de transducteurs ultrasonores (12).

2. Appareil d'imagerie ultrasonore selon la revendication 1, ladite image médicale étant une image du cerveau qui est une quelconque image parmi une image par résonance magnétique (RM), une image par tomodensitométrie (TDM), une image par tomographie par émission de positons (TEP) et une image TEP-TDM capturée respectivement par un dispositif d'imagerie par résonance magnétique (IRM), un dispositif de TDM, un dispositif de TEP et un dispositif de TEP-TDM.

3. Appareil d'imagerie ultrasonore (1) selon la revendication 1, ladite unité de transducteurs (12) comprenant un transducteur à réseau ultrasonore (121) configuré pour effectuer à la fois l'imagerie de l'image du cerveau du patient et la focalisation ultrasonore d'une lésion cérébrale.

4. Appareil d'imagerie ultrasonore (1) selon la revendication 3,
ladite unité de génération d'image (13) étant configurée pour générer une image de diagnostic focalisée par ultrasons sur la base d'un signal ultrasonore de diagnostic reçu du cerveau et
ladite unité d'alignement de position (14) étant configurée pour aligner une position focale de l'image de diagnostic focalisée par ultrasons générée avec une position d'une lésion dans l'image médicale obtenue.

5. Appareil d'imagerie ultrasonore (1) selon la revendication 1, ladite unité de transducteurs (12) comprenant un transducteur d'imagerie (124) configuré pour transmettre et recevoir un signal ultrasonore de diagnostic pour l'imagerie d'une image du cerveau du patient et un transducteur de traitement (123) configuré pour transmettre un signal ultrasonore focalisé à une lésion cérébrale.

6. Appareil d'imagerie ultrasonore (1) selon la revendication 5, ledit transducteur d'imagerie étant un transducteur transcrânien d'imagerie en réseau à commande de phase.

7. Appareil d'imagerie ultrasonore (1) selon la revendication 5, ladite unité de génération d'image (13) étant configurée pour générer une image ultrasonore transcrânienne sur la base d'un signal ultrasonore de diagnostic reçu du cerveau et ladite unité d'alignement de position étant configurée pour aligner une position de l'image ultrasonore transcrânienne générée avec une position d'une lésion dans l'image médicale obtenue.
